# EUROPEAN PATENT APPLICATION

(11) **EP 1 285 577 A1**
(43) Date of publication of application: **26.02.2003**
(21) Application number: 01930151.4
(22) Date of filing: 15.05.2001
(51) Int. Cl.: A01K 67/027, C12P 21/08, C07K 16/00

(54) **CHIMERIC MOUSE HAVING AN IMMUNE SYSTEM CONSTRUCTED WITH HUMAN CD34-POSITIVE CELLS AND USE THEREOF**

(30) Priority: 15.05.2000 JP 2000147467; 17.07.2000 JP 2000221069
(71) Applicant: Habu, Sonoko, Tama-shi, Tokyo 206-0012 (JP)
(72) Inventor: HABU, Sonoko, Tama-shi, Tokyo 206-0012 (JP); ANDO, Kiyoshi, Chigasaki-shi, Kanagawa 253-0037 (JP); HOTTA, Tomomitsu, Tenpaku-ku, Nagoya-shi, Aichi 468-0015 (JP)
(74) Representative: VOSSIUS & PARTNER
(86) International application number: JP0104034
(87) International publication number: WO01087058

(57) **Abstract**

Chimeric mice were constructed by transferring human CD34⁺ cells (hematopoietic stem cells) into a SCID mouse. In these chimeric mice, hematopoietic stem cells persistently differentiated into immune cells. Consequently, the chimeric mice can be immunized over a long time and enable one to obtain human antibodies against arbitrary antigens containing a human self-component.

## Description

### Technical Field

This invention relates to chimeric mice capable of producing human antibodies, methods for producing the human antibodies using the chimeric mice, and the human antibodies prepared by the methods.

### Background Art

Since Köhler and Milstein established the cell fusion technology in 1975 (Köhler, Nature 256: 495-497 (1975)), a variety of monoclonal antibodies have been made and used to measure a variety of samples and to diagnose and treat diseases. The original monoclonal antibodies were prepared in most cases from non-human animals, especially from mice, and, thus, when they were used as therapeutic agents for treating diseases in humans, there was concern about the immunogenicity of the antibodies and their short half-life in the blood. In particular, when they were administered for a chronic disease, frequent administration was required. Thus, application of the antibodies to therapeutic agents was limited. Later, to reduce the immunogenicity, a chimeric antibody comprising the variable region of a mouse antibody and the constant region of a human antibody was made, and further, a humanized antibody, which is obtained by replacing into a human antibody only the complementarity determining region (CDR), essential for antigen-binding activity, was developed. However, the lowest antigenicity can be achieved by an antibody derived from human antibody producing cells. Accordingly, a human monoclonal antibody, a homogeneous human antibody, is useful in the field of therapeutic agents.

There is a known method for producing a human monoclonal antibody in which human antibody producing cells are isolated from a human having a desired antibody in the blood and then immortalized to obtain cell clones producing the human antibody (Unexamined Published Japanese Patent Application No. Hei 5-25058). However, it is difficult to arbitrarily obtain a desired human antibody by the method because it requires the step of isolating a cell producing an antibody possessing a desired activity.

Recently, novel technologies for producing human monoclonal antibodies have been reported (WO92/03918, WO93/02227, WO94/02603, WO94/25585 and WO96/33735), in which transgenic mice comprising a repertoire of human antibody genes are generated and immunized with antigens to obtain mouse cells that produce an antigen-specific human antibodies, which were then immortalized by fusion with myeloma cells and such. However, generation of the transgenic mice described in the gazette requires a large amount of time and effort and is difficult. Moreover, the antibodies produced by the methods comprise sugar chains from mice as described in the following.

In another approach, attempts have been made to generate a chimeric mouse comprising human lymphocytes and to produce an antigen-specific human antibody by immunizing the chimeric mouse with the antigen. Therein, a severe combined immunodeficiency disease (SCID) mouse is used as a host because the mouse does not frequently develop rejection and is incapable of producing mouse antibodies. The SCID mouse was discovered as one having an extremely low concentration of immunoglobulin in the blood, among the C.B.-17 mice, immunoglobulin heavy chain allotype-congenic mice of the Balb/c mice (Nature 301: 527 (1983)). The mouse develops a severe immunodeficiency and is known to be deficient in mature T cells and B cells, the major cells responsible for the immune system (J. Immunol. 132: 1084 (1984)). For the maturation of T cells and that of B cells, the expression of a T cell receptor and that of membrane-bound immunoglobulin molecule, respectively, are required. However, it has been shown that in the SCID mice, a group of enzymes (recombinases) responsible for the rearrangement of the genes essential for the expression of the above molecules are abnormal, and more particularly, the substrate specificity of the recombinase have defects (J. Immunol. 134: 227 (1985)). Therefore, T cells and B cells in the SCID mice are blocked in a virtually immature status, and barely produce any antibodies, not only those against foreign antigens but also those against self-components. As a result, antibody-dependent cellular cytotoxicity (ADCC) is also not observed in the mouse. On the other hand, the functions of antigen presenting cells (APC) and NK cells are normal (Proc. Natl. Acad. Sci. USA 83: 3427 (1988); Cell 55: 7 (1988)).

The aforementioned features of the SCID mice have been utilized to reconstitute the human immune system by transplanting various tissues from animals of different species, in particular by transplanting human lymphocytes and such. For instance, there are reports on the SCID-hu mouse in which histologically intact fragments of human embryonic thymus and liver were transplanted under the renal capsules of the SCID mice (Nature 251: 791 (1991)), and also the hu-PBL-SCID mice, in which human peripheral blood lymphocytes (PBL) were intraperitoneally transplanted (Science 247: 564 (1990)). The hu-PBL-SCID mice were reported to be capable of inducing human specific antibodies against diphtheria-tetanus toxoid and the hepatitis B virus C antigen (J. Exp. Med. 173: 147 (1991)).

However, because the peripheral blood lymphocytes are already differentiated, they have short lifetime, and chimeric mice transplanted with those cannot establish long term immunity. Moreover, the lymphocytes have a defect such that the mouse is not capable of producing an antibody against a human component (autoantibody) because peripheral blood lymphocytes are differentiated mature cells that are already self-adapted.

Moreover, the antibody produced by the methods is in most cases an antibody of IgM class; therefore, it is difficult to produce by inducing affinity maturation an antibody of IgG class with higher binding affinity. For the use as a pharmaceutical agent, it is desired to obtain an IgG class antibody because of the feasibility of production and purification.

### Disclosure of the Invention

The present invention was developed considering the above circumstances, and an objective of the present invention is to generate chimeric mice capable of producing any antibody of interest comprising a human autoantibody and capable of maintaining immunity for long periods and to prepare human antibodies using these mice.

More specifically, the present invention provides chimeric mice having a human immune system that is constructed by transplanting human CD34⁺ cells into SCID mice, methods for preparing human antibodies using the chimeric mice, and human antibodies prepared by the methods.

In a preferred embodiment, this invention provides chimeric mice comprising human mature T cells and mature B cells that are generated by transplanting human CD34⁺ cells collected from human umbilical cord blood into SCID mice. Furthermore, in another preferred embodiment, the present invention provides chimeric mice that are induced to produce an IgG antibody.

Hematopoietic stem cells are pluripotent cells capable of developing and differentiating into all hematopoietic cells; all lymphocytes, including B cells, T cells, and such are also derived from the hematopoietic stem cells. Taking into account the features of those cells, the present inventors presumed that it was possible to solve the problems of the conventional methods by transplanting human hematopoietic stem cells, rather than peripheral lymphocytes, into the mice. Specifically, the present inventors hypothesized that (1) because a chimeric mouse into which the human hematopoietic stem cells were transplanted would persistently develop and differentiate into human T cells and B cells in its body, it could be more persistently immunized than mice produced by the conventional method, using short-lived peripheral lymphocytes, and (2) because the human T cells and B cells matured and differentiated in the mouse body, the mouse could produce an antibody against human components, whereas the conventional methods involving the transfer, into mice, of peripheral lymphocytes that adapted to a human body to be matured and differentiated were unable to do so.

According to such idea, the present inventors generated a chimeric mouse into which human hematopoietic stem cells are transplanted. First, the CD34⁺ cells, a cell population comprising the human hematopoietic stem cells, were prepared from human umbilical cord blood, and transferred into the tail vein of a recipient mouse to generate a chimeric mouse. A NOD-SCID mouse was selected as a recipient mouse because it is incapable of producing mouse antibodies and, owing to a reduced activity of NK cells, is less likely to cause rejection. The present inventors immunized the resultant chimeric mouse with an antigen and examined the ability of the mouse to produce a human antibody. As a result, they found that the chimeric mouse produced antigen-specific human IgM and IgG.

Furthermore, the present inventors transplanted, under both renal capsules of a NOD-SCID mouse, the cells prepared by hybrid reaggregation method (reaggregate thymic organ culture (RTOC) method) for human CD34⁺ cells and mouse thymus stromal cells, and found that the mouse could induce mature T cells from human CD34⁺ cells in its body. Thus, they succeeded in generating a chimeric mouse comprising a fully reconstituted human immune system.

Since the chimeric mouse has mature B cells and mature T cells differentiated from human immature cells, it is possible using the mouse to prepare an antibody against any antigen, including a human self-component. It is also possible to efficiently produce an IgG antibody, inducing the antibody class switch by stimulating the chimeric mouse or the immunocompetent cells, such as spleen cells from the chimeric mouse, with a helper factor, such as human CD40 ligand.

The present invention has been accomplished based on the above findings, and thus provides chimeric mice that are constructed by transplanting human CD34⁺ cells into a SCID mouse and that are capable of producing a human antibody; methods for preparing a human antibody using the chimeric mice; and human antibodies prepared by the methods.

More specifically, this invention provides,
(1) a method for generating a chimeric mouse capable of producing a human antibody, the method comprising transplanting human CD34⁺ cells into a SCID mouse;
(2) the method according to (1), further comprising transplanting, into the SCID mouse, CD34⁺ cells prepared by hybrid aggregation method, in addition to transplanting the human CD34⁺ cells;
(3) the method according to (1) or (2), wherein the human CD34⁺ cells are derived from human umbilical cord blood;
(4) a chimeric mouse generated by the method according to any one of (1) to (3);
(5) a chimeric mouse capable of producing a human antibody, the chimeric mouse comprising mature B cells and mature T cells derived from human;
(6) the chimeric mouse according to (5), wherein the chimeric mouse is generated by the method according to any one of (1) to (3);
(7) a chimeric mouse that persistently carries human T cells and/or B cells derived from human CD34⁺ cells;
(8) a method for preparing a human antibody comprising the steps of:
   (a) immunizing, with an antigen, the chimeric mouse according to any one of (4) to (7) or lymphocytes prepared from the chimeric mouse, and
   (b) recovering a human antibody that binds to the antigen and that is produced by the immunizing of step (a);
(9) the method according to (8), wherein a compound that activates CD40 is administered to the chimeric mouse or contacted with lymphocytes in step (a);
(10) a human antibody prepared by the method according to (8) or (9); and,
(11) the antibody according to (10), wherein the antibody belongs to IgG class.

Herein, the term "human CD34⁺ cells" refers to a population of cells carrying CD34 as a cell surface antigen, the population comprising hematopoietic stem cells. Also, herein, the term "chimeric mice capable of producing a human antibody" refers to mice capable of producing, as a consequence of administration of an antigen, a human antibody that binds to the antigen.

Herein, such a chimeric mouse, capable of producing the human antibody, is generated by transplanting human CD34⁺ cells into a SCID mouse.

The source of CD34⁺ cells is not limited, but those prepared from human umbilical cord blood are preferably used. In the latter case, human CD34⁺ cells may be those immediately separated from human umbilical cord blood, or those once cultured and frozen-stocked. The culture may be performed using a mouse bone marrow stromal cell line (such as HESS-5 cells) as a feeder cell, with human SCF, human TPO, and human F1-2 added (Experimental Hematology 27: 904 (1999)). The molecules are preferably added at around 50 ng/ml. Human CD34⁺ may be prepared using a commercial kit as described in Example 1.

Herein, a standard SCID mouse can be used to transplant human CD34⁺ cells. However, if such a mouse is used, the mouse may cause NK cell-based cytotoxicity against the transplanted CD34⁺ cells, thereby lowering the engraftment ratio of transplanted cells. In this invention, to prevent the reduction in the engraftment ratio of such transplanted cells, NOD-SCID mice, which are SCID mice whose NK cells have reduced activity, are preferably used.

SCID mice and NOD-SCID mice are known in the literature (Nature 301: 527 (1983); J. Immunol. 154: 180 (1995)), and available from suppliers of experimental animals (for instance, Jackson Laboratory) .

For further reducing the activity of NK cells, it is also effective to administer to the mice an antibody specific to NK cells. Examples of antibodies specific to NK cells include anti-asialo GM1 antibody, but are not limited thereto.

Moreover, for improving the engraftment ratio of human CD34⁺ cells, it is also effective to transplant human peripheral blood lymphocytes irradiated with X-rays (preferably around 15 Gy) as accessory cells in the transplantation of the CD34⁺ cells. The number of accessory cells for the transplantation is preferably the same as the number of transferred CD34⁺ cells. Human peripheral blood lymphocytes may be derived from the same donor as that of CD34⁺ cells or from a different donor.

In the method for transplanting human CD34⁺ cells or accessory cells into mice, there is no limitation on the route of transplantation so long as the method enables the transfer of those cells into the blood stream; however, injection through the tail veil is preferably used because it is easily manipulated.

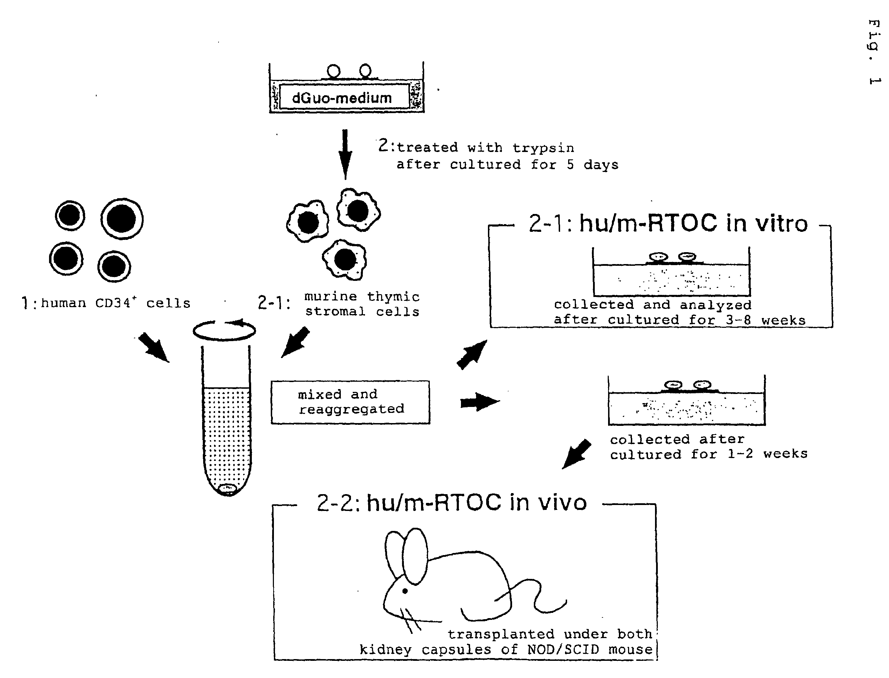

To efficiently produce a human antibody in the chimeric mouse, it is preferable that both B cells and T cells in the chimeric mice are derived from human. For generating such chimeric mice, in which human immune system is fully reconstituted, human CD34⁺ cells may be co-transplanted into a recipient mouse with further differentiated human CD34⁺ cells by hybrid aggregation method (RTOC method) (Immunol. Letter, 71: 61 (2000); J. Exp. Med., 176: 845 (1992)). In RTOC method, for example, after mouse fetal thymus is treated with deoxyguanosine (dGuo), the epithelial cells of the thymus are collected and re-aggregated with human CD34⁺ cells, which are subsequently cultured for about a week to generate hybrid aggregate of the human CD34⁺ cell with mouse epithelial cells to be transplanted into recipient mice. The chimeric mice comprising a complete human immune system can be constructed not only by transferring the human CD34⁺ cells into SCID mice via the tail vein, but also by transplanting the human CD34⁺ cell aggregate thus prepared by hybrid aggregation method under the renal capsules of SCID mice. Fig. 1 shows schemes for performing the hybrid aggregation method using human CD34⁺ cells and mouse thymic epithelial cells.

To efficiently produce human antibodies in chimeric mice, in addition to the method of transferring human CD34⁺ cells further differentiated into mature T cells by the RTOC method, for example, administration of soluble factors derived from human T cells may be substituted in the role of human T cells. For instance, human CD40 ligand (hCD40L) may be used as the T cell-derived factor. The successful obtaining of IgG class antibody-producing cells by adding hCD40L, IL-4, and IL-10 to *in vitro* culture has been reported (Blood 92: 4501 (1998)). It has also been reported that, when SCID mice into which human peripheral blood is transplanted are immunized with diphtheria-tetanus toxoid (DT), not only IgM-class but also IgG-class anti-DT antibodies may be obtained by administering anti-CD40 antibody together with DT (Clinical Immunol. 90: 4632 (1999)). Moreover, it has been reported that the administration of an anti-CD40 antibody together with a T cell-independent antigen results in the occurrence of an antigen-specific IgG antibody in mice (Nature Medicine 4: 88 (1998)). Therefore, it is possible to more efficiently yield the IgG-producing cells by activating CD40, either through transplanting transformed cells producing hCD40L or by injecting hCD40L or an anti-hCD40 antibody into mice. For example, hCD40L (2 µg) or anti-CD40 antibody (2 µg) may be injected every other day, 10 times in total. Alternatively, an anti-CD40 antibody may be injected 10 times in total, preferably at 1 to 50 µg/head, more preferably at 5 to 30 µg/head, and most preferably at 10 to 20 µg/head.

A transformed Hela cell producing hCD40L may be used as a source for the purification of the molecule, for example. In addition, purified monoclonal antibody derived from a mouse hybridoma cell line (5C3) may be used as an anti-hCD40 antibody, for example.

The chimeric mice generated by the above mentioned method are capable of producing, by administering an antigen thereto, a human antibody that is capable of binding to the administered antigen. The method for administering the antigen and recovering the human antibody produced in the mice is known to one skilled in the art (see Example 3).

For preparing a human antibody, in addition to directly immunizing the chimeric mice of the present invention with an antigen, lymphocytes prepared from the mice may be also sensitized with the antigen *in vitro. In vitro* sensitization with an antigen may be performed according to known methods (Arai, Experimental Medicine, 6: 897-903 (1988)). The lymphocytes used for the *in vitro* sensitization may be derived from, for example, spleen cells.

To efficiently produce an IgG antibody *in vitro,* it is also effective to expand the cell clones producing an antigen-specific antibody and to induce class switching by using a helper factor. Specifically, for instance, 7 days after hu-SCID mice (chimeric mice into which human CD34⁺ cells are transplanted) are once immunized with an antigen, the spleen cells are collected and re-stimulation is performed by adding the antigen to the culture together with a helper factor *in vitro*. Exemplary helper factor include soluble hCD40L, IL-4, or IL-10. These helper factors are preferably added to the culture at a concentration of about 10 µg/ml for soluble hCD40L, and about 0.5 to 50 ng/ml for IL-4 and IL-10.

The titer and the class of the antibody secreted into the culture supernatant can be evaluated by ELISA, by adding samples to the plates coated with the antigen, and detecting the signal using a labeled antibody against each class of human immunoglobulin.

### Brief Description of the Drawings

Fig. 1 depicts a scheme for performing the hybrid aggregation method using human CD34⁺ cells and mouse thymic stromal cells.

Fig. 2 depicts titers of antibodies specific to human antigens in the serum from NOD-SCID mice. The Y-axis indicates the titer of DNP-specific antibodies in the sera collected each week.

Fig. 3 depicts the amount of IgM- and IgG-class antibodies in the anti-DNP-KLH antibody. The Y-axis indicates the amount of antibody (ng/ml).

Fig. 4 depicts the differentiation and induction of T-cell function (ability of producing IL-2) from human CD34⁺ cells. (A) shows the results for culture of lymphocytes differentiated *in vitro* by human-mouse hybrid aggregation method. (B) shows the results for culture of lymphocytes differentiated *in vivo.* The X-axis indicates the number of weeks after transplantation, and the Y-axis indicates the amount of IL-2 produced (pg/ml).

### Best Mode for Carrying out the Invention

This invention is explained in detail below in examples, but should not to be construed as being limited thereto.

### [Example 1] Isolation of CD34⁺ cells from human umbilical cord blood:

Human umbilical cord blood was collected, overlaid on top of Ficoll-Hypaque, a hemocyte separating media (d = 1.077; Amersham Pharmacia), and centrifuged at 2,000 rpm for 30min at 20°C. The leukocyte phase, comprising separated lymphocytes at the interface between two separated phases, was collected, and washed three times with PBS containing 1% BSA and 0.02% EDTA (Washing buffer). The resulting cellular fraction of leukocytes was separated using the MACS CD34 immunomagnetic isolation kit (Miltenyi Biotec, Glodbach, Germany), and CD34⁺ cells were thus obtained. Specifically, the cells were labeled with magnetic beads according to the manufacturer' s instruction and washed with the washing buffer. The VS+ column was mounted onto the MACS separator, and CD34⁺ cells were separated. The collected cells were positively selected on the RS column again. After the number of the separated cells was counted, the solvent was replaced with PBS, and the cells were used in the following manipulations.

### [Example 2] Generation of a mouse into which human lymphocytes is transplanted:

At the age of 8 weeks, NOD/sci-scid (NOD-SCID) mice (J. Immunol. 154: 180 (1995)) were irradiated with X-rays at 3.5 Gy, which is a 50% lethal dose, and the human CD34⁺ cells prepared according to Example 1 were transplanted via the tail vein into the mice at 500,000 cells/mouse. To improve the engraftment ratio of transplanted cells, human peripheral blood lymphocytes that had been irradiated with X-rays at 15 Gy were transferred into the tail vein as accessory cells, at 500,000 cells/mouse. Furthermore, for the same purpose, 10 ml of anti-asialo GM1 antibody (Wako Jyunyaku) was intraperitoneally injected on the day before transplantation, the day of transplantation, and two days after transplantation, in order to reduce the activity of NK cells derived from the mice. Four weeks later, human SCF (20 µg/kg/day) (Amgen Biologicals) and G-CSF (25 µg/kg/day) (Kirin) were intraperitoneally injected for 4 days.

### [Example 3] Sensitization with an antigen and measurement of an antibody:

Six week after transplantation of human CD34⁺ cells, a T cell-independent (TI) antigen, Ficoll-DNP (J. Immunol. 114: 704 (1975)) (50 µg/head), or a T cell-dependent (TD) antigen, KLH-DNP or OVA-DNP (Methods Med. Res. 10: 94 (1964)) (25 µg/head), was mixed with an equal volume of the complete Freund's adjuvant (Difco Laboratories), and intraperitoneally injected. Mice were immunized every two weeks in the same way, with the exception that the incomplete Freund's adjuvant (Difco Laboratories) was used as the adjuvant. After immunization was initiated, blood samples were collected every week suborbitally, and the titer of the antibody originating from the transplanted human cells and the frequency of occurrence of human T cells and B cells in the peripheral blood were examined. The titer was examined by ELISA using the serum separated from the collected blood samples and plastic plates coated with KLH-DNP. Specifically, the plates were coated with DNP conjugated to a carrier and blocked in 3% BSA at room temperature for 2 hr. The 10- to 50-fold diluted serum was added to the wells at 100 µl/well, and reaction was performed at room temperature for 2 hr. The wells were washed with the rinsing buffer, and a biotin-conjugated anti-human IgM or IgG monoclonal antibody, diluted at 1:3000, was added to the wells at 100 µl/well to react at 37°C for 2 hr. After washing, avidin-conjugated peroxidase, diluted at 1:5000, was added at 100 µl/well and reacted at room temperature for 1 hr. After washing, the resulting complex was subjected to color developing using the TMB peroxidase EIA substrate kit (BioRad) by incubating at room temperature for 30 min. The reaction was terminated with 10% HCl, and the absorbance at 450 nm was measured.

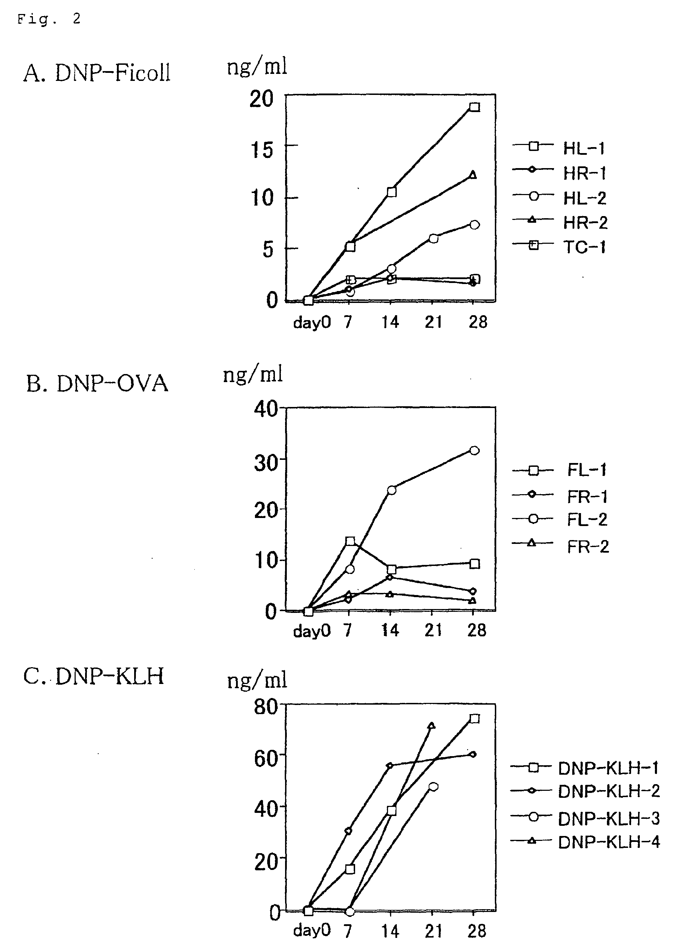

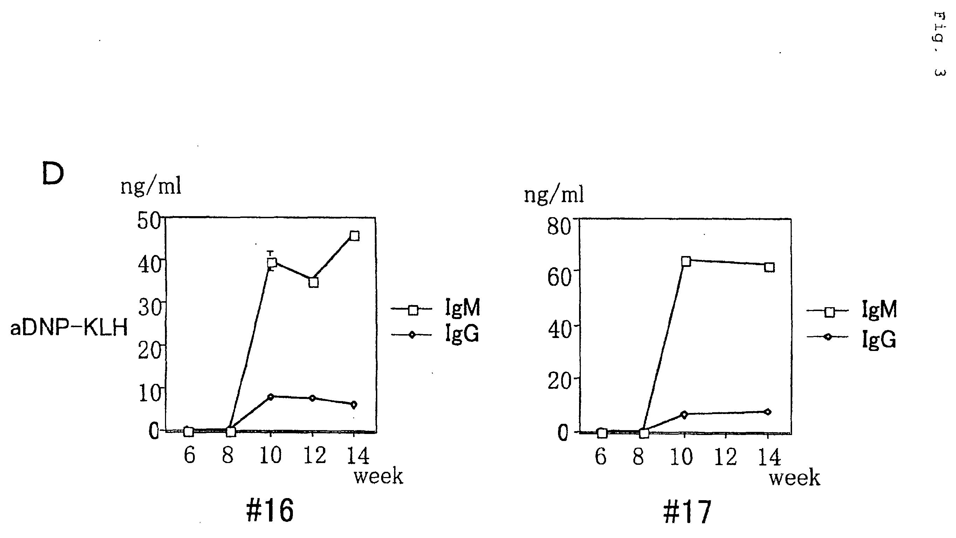

The result showed that a DNP-specific antibody was detected in the three out of five mice immunized with DNP-Ficoll, a T independent antigen (TI) , and that one of the mice showed a extremely high antibody titer (Fig. 2A). Among the mice immunized with DNP-OVA or DNP-KLH, a TD, one out of four mice immunized with DNP-OVA and all four mice immunized with DNP-KLH showed high DNP specific antibody titer (Fig. 2B and C) . In addition, antibodies of IgG class as well as IgM class were detected in the anti-DNP-KLH antibodies (Fig. 3).

### [Example 4] Examination of the frequency of occurrence of human T cells and B cells:

The frequency of the occurrence of human T cells and B cells was examined by flow cytometry (FACS) using the lymphocytes fractions prepared from the peripheral blood sample using Ficoll (Pharmacia) and stained with a variety of antibodies directed against specific antigens for human T cells and B cells. For B cells, PE-anti-CD19 antibody, a B cell marker, was used in conjunction with FITC-anti-CD5 antibody, FITC-anti-IgM antibody, FITC-anti-IgG antibody, or FITC-anti-CD40 antibody, and the presence of subsets of B cells and the degree of differentiation were analyzed. For analyzing T cells, PE-anti-CD2 antibody, FITC-anti-CD3 antibody, and FITC-anti-CD4 antibody were used (all antibodies were from Becton Dickinson). The reaction was performed at 0°C for 20 min. Cells were washed with the FACS buffer, suspended in 0.5 ml of the FACS buffer, and analyzed by the FACScan (Becton Dickinson) for the fluorescence intensity reflecting the amount of the antibody reacting with the each cell. In the analysis, CD45⁺ cells were gated and the ratio of cells carrying the T cell marker or the B cell marker could be calculated.

The results showed that the percentage of human B cells (CD45⁺ cells) in all leukocytes was approximately 30% in spleen, 40% in bone marrow, and 2% in peripheral blood in the NOD-SCID mice into which human CD34⁺ cells were transplanted. On the other hand, the percentage of the cells positive for the human T cell marker was below the detection limit in the FACS analysis. The cells expressing a mouse T cell marker or mouse B cell marker were detected around 1 to 2%.

### [Example 5] Induction of differentiation into T cell from human umbilical cord blood:

Thymus was excised from BALB/c mice embryo at the age of 15 days, and a fetal thymic organ culture (FTOC) was performed on the nucleopore Track-Etoh Membrane (Corning) in the presence of 1.3 mM deoxyguanosine (dGuo; Sigma) for 4 days, and both lymphocytes and dendritic cells were removed. The thymus tissue was cultured for an additional day in the dGuo-free medium, and then treated with PBS containing 0.25% trypsin (Sigma) and 0.02% EDTA (Wako Jyunyaku). Thus, released thymic epithelial cells (stromal cells) were obtained. The stromal cells were mixed with human CD34⁺ cells at 1:4 ratio and centrifuged at 2000 rpm. The resulting human/mouse hybrid reaggregate (hu/m hybrid) was cultured on the nuclepore Track-Etoh Membrane for 2 weeks (RTOC). After 2-week RTOC, the hu/m hybrid was transplanted under the renal capsule of NOD-SCID mice. The transplanted hu/m hybrid was analyzed by FACScan for the differentiation status of human T cells using T cell-specific antibodies (anti-CD1a, anti-CD4, anti-CD8, anti-CD3, and anti-CD45 antibodies).

The results revealed that the human CD34⁺ cells cultured by RTOC differentiated into mature T cells that were positive for both CD4 and CD8.

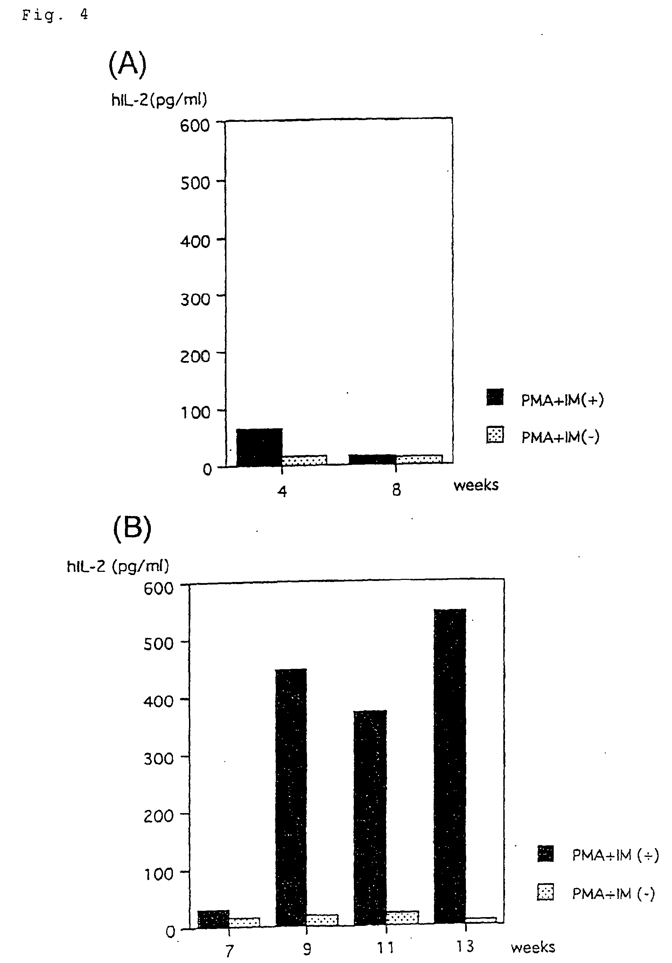

Although the *in vitro* cultured human CD34⁺ cells hardly induced the functional differentiation, such as cell proliferation, IL-2 production, or such (Fig. 4A), those obtained by transplanting the cultured aggregate into the renal capsule of NOD-SCID mice following RTOC showed a remarkable increase in cell proliferation and acquisition of IL-2 production ability (Fig. 4B). Thus, the results confirmed the previous reports showing that thymic stromal cells were capable of inducing differentiation of T cells beyond species, and furthermore, it demonstrated for the first time that human CD34⁺ cells were capable of functionally differentiating into T cells under more physiological conditions such as *in vivo.*

In this example, the IL-2 production was assayed using the ELISA kit and determining the concentration of hIL-2 produced in the culture supernatant of the cells stimulated with phorbol myristate acetate (PMA) and IM (ionomycin). Specifically, the reaggregate transplanted under the renal capsule was sterilely excised, and passed through a nylon mesh to remove the undesired aggregates of cells and dead cells. Thus, a cell suspension comprising primarily lymphocytes was prepared. The cells were suspended in RPMI culture media at 5x10⁶ cells/ml, and plated onto round-bottom 96 well plates at 100 µl/well. PMA (final concentration: 20 ng/ml) plus IM (final concentration: 200 ng/ml) was added to the culture in each well incubated for 24 hr at 37°C, and the culture supernatant was collected. The assay was performed using an ELISA kit for measuring IL-2 concentration (Endogen Human Interleukin-2 ELISA kit).

### [Example 6] Production of a human IgG antibody using an anti-human CD40 antibody:

The mice into which human CD34⁺ cells were transplanted were generated according to the method described in Example 2, and after 8 weeks, the mice were not only intraperitoneally administered DNP-KLH (100 mg) (first immunization) but also subcutaneously administered an anti-human CD40 antibody (20 µg) (5C3: Pharmingen). The anti-human CD40 antibody (20 µg) alone was further administered every other day, in a total 10 administrations, until the 11th week. At the 11th week, DNP-KLH was intraperitoneally administered for the second time (booster) . At the 12th week, the spleen was excised from the mice under anesthesia, and used for (1) the identification of human lymphocytes in the peripheral lymphatic tissues, and (2) the detection of an antigen specific human antibody in the mouse peripheral blood and the spleen cell culture by ELISA. The identification of human lymphocytes in the peripheral lymphonodes was performed according to the method described in Example 4, and the detection of an antigen specific human antibody was performed according to the method described in Example 3.

The results showed that: (1) while there was no change found in the percentage of human B cells in the peripheral blood of the chimeric mice that were administered the anti-human CD40 antibody, the percentage in bone marrow and spleen was increased, and (2) the production of an antigen specific antibody (anti-DNP antibody) was markedly increased by injecting the anti-human CD40 antibody. Therein, the IgM class was the major isotype, and the IgG class showed a tendency to increase. The results indicate that human CD34⁺ stem cells differentiated into B cells in the mouse peripheral tissues and expanded at clonal level. They also demonstrated that injection of an anti-human CD40 antibody was an effective way not only to promote the proliferation of B cell clones capable of producing an antibody, but also to produce an antibody of the IgG class.

### Industrial Applicability

The present invention provides chimeric mice that are generated by transplanting human CD34⁺ cells and that are capable of producing human antibodies, the methods for generating the mice, and the methods for preparing the human antibodies using the chimeric mice.

Unlike the conventional chimeric mice generated by transplanting human peripheral blood lymphocytes, the chimeric mice of the present invention harbor undifferentiated hematopoietic stem cells transplanted thereinto and further harbor lymphocytes that are differentiated in their body. These lymphocytes are not self-adapted (not adapted to humans), contrary to the human peripheral blood lymphocytes used in the conventional methods. Therefore, the chimeric mice of the present invention are able to produce human antibodies against any antigens, including those with a human self-component.

Moreover, human CD34⁺ cells do not express the receptor for EBV (Epstein-Barr virus), and, thus, there is no need to worry about a contamination of EBV when utilizing the chimeric mice of the present invention into which human CD34⁺ cells are transplanted.

Moreover, whereas it is known that the dendritic cells present in the peripheral blood lymphocytes are not so potent in presenting an antigen as to trigger an effective immune response, the transplanted hematopoietic stem cells in the chimeric mice of the present invention stably proliferate and differentiate into immunocompetent cells such as B cells, T cells, and dendritic cells, which can provide a long term immunization.

Furthermore, the antibodies of the present invention comprise a complete human immunoglobulin including sugar chains. The currently known transgenic mice into which a human antibody gene is introduced utilize mouse-derived B cells to produce an antibody, which results in binding of a mouse sugar chain. Thus, the use of such antibodies may lead to the development of anti-mouse antibodies comprising sugar chain when the antibodies are used as pharmaceutical drugs that requires repetitive administration. In contrast, the human antibodies produced by the present invention comprise a human sugar chain, and, thus, are unlikely to cause a problem of generating a neutralizing antibody and thus more useful.

The present invention also enables one to obtain an antibody of IgG class with high avidity by the use of a helper factor. This is extremely important because it provides feasible methods for production and purification of the antibody of the present invention in using the antibodies as a pharmaceuticals.

## Claims

1. A method for generating a chimeric mouse capable of producing a human antibody, the method comprising transplanting human CD34⁺ cells into a SCID mouse.

2. The method according to claim 1, further comprising transplanting, into the SCID mouse, CD34⁺ cells prepared by hybrid aggregation method, in addition to transplanting the human CD34⁺ cells.

3. The method according to claim 1 or 2, wherein the human CD34⁺ cells are derived from human umbilical cord blood.

4. A chimeric mouse generated by the method according to any one of claims 1 to 3.

5. A chimeric mouse capable of producing a human antibody, the chimeric mouse comprising mature B cells and mature T cells derived from human.

6. The chimeric mouse according to claim 5, wherein the chimeric mouse is generated by the method according to any one of claims 1 to 3.

7. A chimeric mouse that persistently carries human T cells and/or B cells derived from human CD34⁺ cells.

8. A method for preparing a human antibody comprising the steps of:
(a) immunizing, with an antigen, the chimeric mouse according to any one of claims 4 to 7 or lymphocytes prepared from the chimeric mouse, and
(b) recovering a human antibody that binds to the antigen and that is produced by the immunizing of step (a).

9. The method according to claim 8, wherein a compound that activates CD40 is administered to the chimeric mouse or contacted with lymphocytes in step (a).

10. A human antibody prepared by the method according to claim 8 or 9.

11. The antibody according to claim 10, wherein the antibody belongs to IgG class.
